# EUROPEAN PATENT SPECIFICATION

(11) **EP 2 498 682 B1**
(45) Date of publication and mention of the grant of the patent: **04.09.2013**
(21) Application number: 10782421.1
(22) Date of filing: 05.11.2010
(51) Int. Cl.: A61B 8/08, A61B 8/00, G01F 23/296, G01F 23/30, G01N 29/22, G01N 29/24

(54) **ACOUSTIC WAVE MEASURING APPARATUS**
SCHALLWELLENMESSER
APPAREIL DE MESURE D'ONDE ACOUSTIQUE

(30) Priority: 12.11.2009 JP 2009258930
(43) Date of publication of application: 19.09.2012
(60) Divisional application: 13178150.2
(73) Proprietor: Canon Kabushiki Kaisha, Tokyo 146-8501 (JP)
(72) Inventor: FUJIMOTO, Akira, Ohta-ku, Tokyo (JP); TOKITA, Toshinobu, Ohta-ku, Tokyo (JP)
(74) Representative: TBK
(86) International application number: PCT/JP2010/006510
(87) International publication number: WO 2011/058724

(56) References cited:
- WO-A1-00/09014
- WO-A1-2005/122904
- WO-A1-2008/151277
- WO-A1-2010/094848
- JP-A- 2004 141 260
- US-A- 4 429 577

## Description

### Technical Field

The present invention relates to an apparatus for measuring an acoustic wave such as an ultrasonic wave, and especially relates to an acoustic wave measuring apparatus used in living body inside observation.

### Background Art

A living body inside observation apparatus for irradiating a subject with an acoustic wave and visualizing a reflective wave thereof, thereby imaging the inside of the subject is conventionally known as a diagnostic apparatus using the acoustic wave. In such living body inside observation apparatus, an acoustic wave probe for emitting the acoustic wave and receiving the reflective wave (echo) to output the signal is used. The acoustic wave received by the probe is converted into an electric signal and an image is generated to be displayed.

Also, recently, a diagnostic system of a photo-acoustic tomography (PAT) using a photo-acoustic effect has been developed. In this apparatus, the subject (breast) is pressed by a compression plate and the acoustic wave probe and the breast is irradiated with illumination light (near-infrared light) of which source is Nd:YAG laser over the compression plate. Then, a photo-acoustic wave generated in the subject (breast) is received by the probe and a tissue in the subject (breast), especially, a new blood vessel in breast cancer is displayed by image reconstruction.

Such apparatus for measuring the acoustic wave generated from the living body interior must have a configuration in which a gap is not generated (air is not mixed) between members through which the acoustic wave transmits in order to ensure acoustic impedance matching.

Fig. 10A illustrates a structure of an ultrasonic wave probe disclosed in Japanese Patent Application Laid-Open No. 2003-325523 (PTL1). In this probe, in order to fill a space between an ultrasonic wave conversion unit 1 and a compression plate 2 with lubricant, a sponge 3 impregnated with the lubricant is provided between the ultrasonic wave conversion unit 1 and a cover 4. According to this, a thin film of the lubricant is formed when the ultrasonic wave conversion unit 1 moves along the compression plate 2, and the ultrasonic wave conversion unit 1 and the compression plate 2 tightly adhere to each other.

Fig. 10B illustrates a structure of the ultrasonic wave probe disclosed in Japanese Patent Application Laid-Open No. 2004-141260 (PTL2). A probe 12 includes an ultrasonic wave conversion unit 5, a storage unit 7 for storing gel, an acoustic lens 9, an ejection port 6, a delivery pipe 10 and a pump 11. The gel in the storage unit 7 is ejected from the ejection port 6 through the delivery pipe 10 by the pump 11. Therefore, a space between the subject and the acoustic lens 9 is filled with the gel and adhesiveness is improved.
Also, a configuration of providing a pump 13 and a storage unit 14 for storing the gel out of the ultrasonic wave probe 12 as illustrated in Fig. 10C is also disclosed in Japanese Patent Application Laid-Open No. 2004-141260. By operation of the pump 13, the gel is provided to the probe 12 through a delivery tube 15.
(PTL1) JP2003-325523A
(PTL2) JP2004-141260A

### Summary of Invention

In the above-described conventional configuration, the adhesiveness might be deteriorated due to insufficiency of the lubricant when the lubricant is not provided well due to insufficiency of remaining lubricant and clog of the delivery pipe. Also, there is a case in which the lubricant flows out when the ultrasonic wave probe is moved, and the sufficient adhesiveness is not obtained. When a gap is generated between the acoustic wave conversion unit and the compression plate during measurement, the reliability of a result of measurement is lowered and this is not preferable. However, a trouble in providing the lubricant could not be detected with the conventional configuration.

The present invention is achieved in view of the above-described circumstances, and an object thereof is to provide a technique of appropriately keeping a state of the lubricant between the acoustic wave conversion unit and the compression plate, thereby improving the reliability of the measurement.

An acoustic wave measuring apparatus according to this invention comprising:
a plate pressed against a subject;
a probe that has an acoustic wave conversion unit for receiving an acoustic wave emitted from the subject through the plate to convert into an electric signal;
a seal member that is provided on the probe so as to enclose a periphery of the acoustic wave conversion unit, and that forms a space to hold lubricant between the plate and the acoustic wave conversion unit;
a providing unit that provides the lubricant into the space formed by the seal member; and
a lubricant monitoring unit that detects whether the space between the plate and the acoustic wave conversion unit is filled with the lubricant.

According to the present invention, the state of the lubricant between the acoustic wave conversion unit and the compression plate may be appropriately kept and the reliability of the measurement may be improved.

Further features of the present invention will become apparent from the following description of exemplary embodiments with reference to the attached drawings.

Documents WO2005122904 and WO0009014 disclose an apparatus according to the pre-amble of claim 1.

### Brief Description of Drawings

[fig.1]Fig. 1 is a block diagram of an acoustic wave measuring apparatus of a first embodiment.
[fig.2]Fig. 2 is a perspective view of the acoustic wave measuring apparatus.
[fig.3]Fig. 3 is a perspective view of an acoustic wave probe of the first embodiment.
[fig.4]Figs. 4A, 4B and 4C are cross-sectional views of the acoustic wave probe.
[fig.5]Fig. 5 is a flowchart illustrating a flow of a premeasurement preparation process.
[fig.6]Fig. 6 is a flowchart illustrating a flow of a monitoring process of lubricant during measurement.
[fig.7]Fig. 7 is a trajectory diagram of the acoustic wave probe during the measurement.
[fig.8]Fig. 8 is a perspective view of the acoustic wave probe of a second embodiment.
[fig.9]Fig. 9 is a block diagram of the acoustic wave measuring apparatus of the second embodiment.

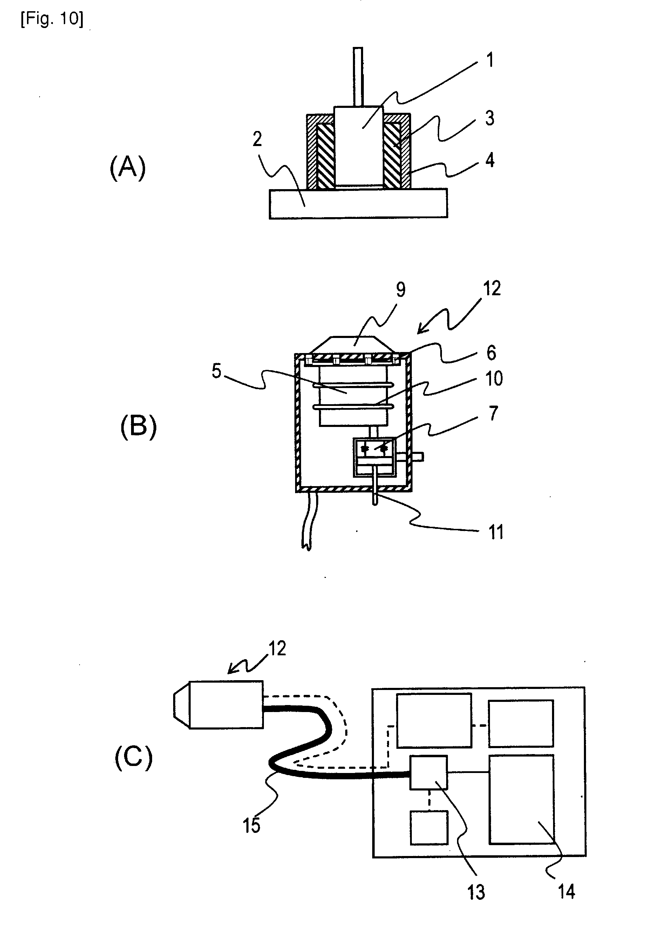

[fig.10]Figs. 10A, 10B and 10C are views illustrating a structure of a conventional ultrasonic wave probe.

### Description of Embodiments

### <First Embodiment>

A preferred embodiment of an acoustic wave measuring apparatus according to the present invention is described with reference to the drawings.

The acoustic wave measuring apparatus of the present invention is an apparatus used for receiving an acoustic wave, which is transmitted in a subject to be emitted from the subject (living body and the like), conversion the same to an electric signal and obtaining information in the living body (a cross-sectional image, optical characteristic value distribution and the like) from the signal. The acoustic wave measuring apparatus may be preferably applied to a living body inside observation apparatus such as an ultrasonic diagnostic apparatus using ultrasonic echo and a photo-acoustic tomography apparatus (PAT), for example. Hereinafter, a configuration of a part, which receives the acoustic wave, is mainly described. Meanwhile, although an ultrasonic wave is typically used as the acoustic wave, an elastic wave in an audible field of which frequency is lower than that of the ultrasonic wave may also be used.

### (Acoustic Wave Measuring Apparatus)

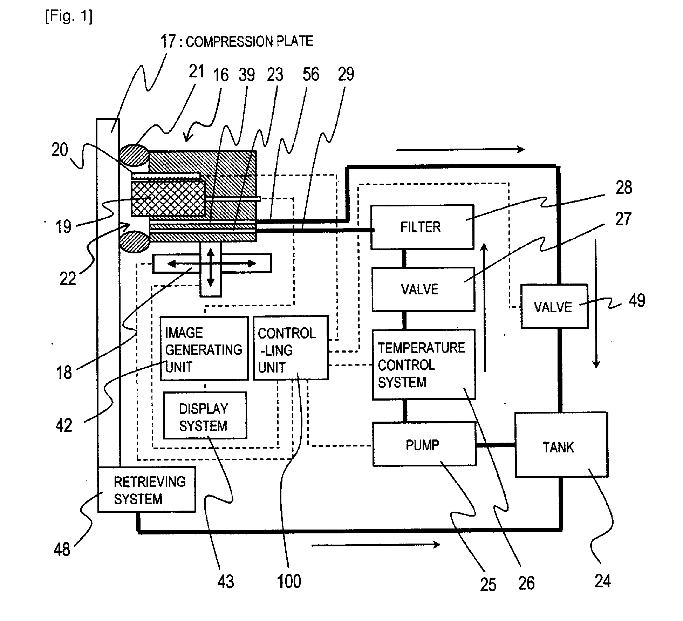

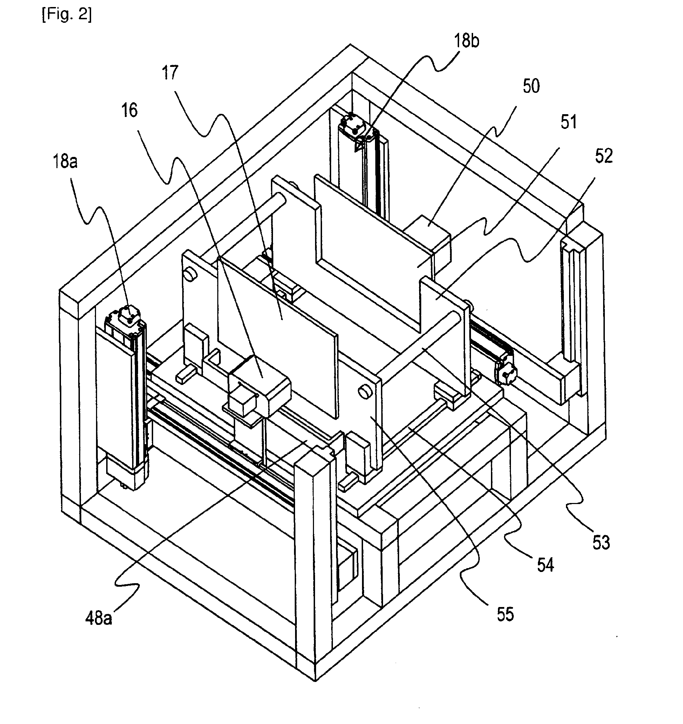

Fig. 1 illustrates a schematic configuration of an acoustic wave measuring apparatus of a first embodiment. Fig. 2 is a perspective view of the acoustic wave measuring apparatus.

In Fig. 1, an acoustic wave probe 16 (hereinafter, also simply referred to as probe 16) is attached to a biaxial drive unit 18 and is two-dimensionally movable along a surface of a fixed compression plate 17. The compression plate 17 is a plate to be pressed against the subject, which is an object to be measured. The acoustic wave probe 16 includes an acoustic sensor for measuring the acoustic wave generated from the subject through the compression plate 17. An acoustic wave conversion unit 19 for conversion the acoustic wave into the electric signal may be used as the acoustic sensor. A transducer using a piezoelectric phenomenon, a transducer using optical resonance, a transducer using a change in capacity and the like may be used as the acoustic wave conversion unit 19 (hereinafter, also simply referred to as conversion unit 19), and a transducer array in which a plurality of transducers is one-dimensionally or two-dimensionally arranged may also be used. In order to ensure acoustic impedance matching from the living body to the acoustic wave conversion unit 19, resin is preferably used as a material of the compression plate 17 and polymethylpentene is especially preferably used.

The probe 16 has a seal member 21, which encloses a periphery of the conversion unit 19. A space 22 for holding lubricant between the compression plate 17 and the conversion unit 19 is formed by the seal member 21. A delivery port 23 for providing the lubricant into the space 22 is formed on the probe 16. The lubricant stored in a tank 24 is delivered by a pump 25, which is a providing unit, through a pipe 29. Deaerated lubricant is preferably used, and oil, water, acoustic gel and the like may be used as the lubricant, for example. In a case of the oil, castor oil is preferable. Polyethylene, stainless and the like are applicable as a material of the tank 24. However, when insulating lubricant is circulated, this may be charged with static electricity, so that a configuration in which an earth cable is attached to the tank made of stainless to discharge the static electricity to ground is preferable. Also, the pipe 29 to deliver the lubricant may be made of polyethylene or stainless; however, a configuration in which the same is made of stainless and the static electricity is discharged to ground through the earth cable is preferable. The tank 24 includes a liquid level monitoring unit not illustrated for sensing decrease of the lubricant due to abnormal leakage. As the unit for monitoring the liquid level, the unit, which may sense also in a case of insulating lubricant, such as a flow-type unit, an optical unit and an ultrasonic unit is preferable.

The lubricant delivered from the tank 24 is provided from the delivery port 23 into the space 22 through a temperature control system 26, a valve 27 and a filter 28. The temperature control system 26 is provided with a function to measure a temperature of the delivered lubricant and a function to heat or cool the lubricant to a predetermined temperature. Therefore, variation in a temperature condition of the lubricant, the acoustic wave conversion unit 19 and the like is supperessed, so that stable measurement becomes possible. The valve 27 is for adjusting a flow amount and a pressure of the lubricant. The filter 28 has a function to remove dust such that the dust mixed during circulation of the lubricant does not enter the space 22. A filter having a mesh structure may be used, for example. Therefore, generation of measurement noise by the dust and damage of the conversion unit 19 and the compression plate 17 may be suppressed.

At the time of the measurement, the space 22 formed by the compression plate 17, the conversion unit 19 and the seal member 21 (at least, a portion between the compression plate 17 and the conversion unit 19) is filled with the lubricant. In the present embodiment, a lubricant monitoring unit provided on the probe 16 detects whether the space 22 is filled with an appropriate amount of lubricant.

The acoustic wave conversion unit 19 may double as the lubricant monitoring unit. When there is no lubricant between the transducer and the compression plate 17, the acoustic wave cannot be received or a reception level of the acoustic wave is significantly lowered. This is used for detecting the lubricant. For example, it is possible to use the transducers of a top row of the acoustic wave conversion unit 19 as the lubricant monitoring unit and determine whether the acoustic wave may be detected by the transducers, thereby determining whether the amount of the lubricant is appropriate. It is also possible to determine that the amount of the lubricant is appropriate (the space 22 is filled with the lubricant) when the acoustic wave of an equivalent level may be detected by all the transducers of the acoustic wave conversion unit 19 and determine that a providing state of the lubricant is not appropriate (lubricant is insufficient) when the reception level of the acoustic wave largely varies from transducer to transducer. When a bubble is mixed in the lubricant, the reception level of the transducer of a part of the bubble falls, so that the acoustic wave conversion unit 19 may be used for monitoring the bubble.

Alternatively, a dedicated sensor as the lubricant monitoring unit may be provided in addition to the acoustic wave conversion unit 19. In an example illustrated in Fig. 1, a liquid level sensor 20 is arranged above the conversion unit 19. The liquid level sensor 20 detects whether the space 22 is filled with the lubricant to a position higher than the acoustic wave conversion unit 19. As the liquid level sensor 20, any type of sensor such as an optic sensor, an ultrasonic sensor, a capacitance sensor and a float-type sensor may be used, and an appropriate type may be selected according to a type of the lubricant (for example, the optic sensor and the ultrasonic sensor are preferable for the insulating lubricant).

A retrieving system 48 as a retrieving unit for retrieving the lubricant, which leaks out of the space 22 from the seal member 21 is provided on a lower part of the compression plate 17. The retrieving system 48 of the present embodiment is composed of a receiving container 48a (refer to Fig. 2) for receiving the lubricant running down the compression plate 17 and a pipe for connecting the receiving container 48a and the tank 24. During the measurement, the lubricant, which leaks from the seal member 21 is retrieved by the retrieving system 48 and is allowed to circulate to the tank 24. Meanwhile, a shape and a volume of the space 22 are designed such that the lubricant with which the space between the compression plate 17 and the conversion unit 19 is sufficiently filled remains from start to end of the measurement even when the lubricant leaks in association with the movement of the probe 16. Specifically, it is preferable that a maximum amount of the lubricant, which leaks while the probe 16 moves from a start position to an end position of the measurement, is estimated by experiment or calculation, and the shape and the volume of the space 22 are designed such that the liquid level of the lubricant is higher than the conversion unit 19 even when the amount is subtracted. After the measurement, the lubricant in the space 22 is retrieved from a retrieve port 39 through a pipe 56 to the tank 24 by opening a valve 49.

As illustrated in Fig. 2, two compression plates 17 and 51 are arranged in parallel. The compression plate 17 is fixed to a fixing plate 55 and the compression plate 51 is fixed to a fixing plate 52. The fixing plates 55 and 52 are provided so as to be movable in parallel by a guide rod 53 and a linear guide 54. A measurement site of the subject is interposed between the compression plates 17 and 51, and an interval between the compression plates 17 and 51 is controlled by a compression mechanism not illustrated, such as a mechanism by a trapezoidal thread and a bevel gear and an air cylinder mechanism, for example, to hold the measurement site therebetween. When only transmitting and receiving the acoustic wave, the probe 16 is moved along the compression plate 17 by a drive unit 18a. A configuration of the drive unit 18a may be that composed of a motor, a ball screw and a guide, that composed of the motor, a gear and a belt link and that composed of an air cylinder and the guide. When measuring using a photo-acoustic effect, a light source 50 for emitting pulse light is attached to a drive unit 18b. By synchronously controlling the drive units 18a and 18b, the light source 50 and the acoustic wave probe 16 are oppositely arranged across the subject and the acoustic wave generated in the subject by the light emitted from the light source 50 is measured by the probe 16.

### (Acoustic Wave Probe)

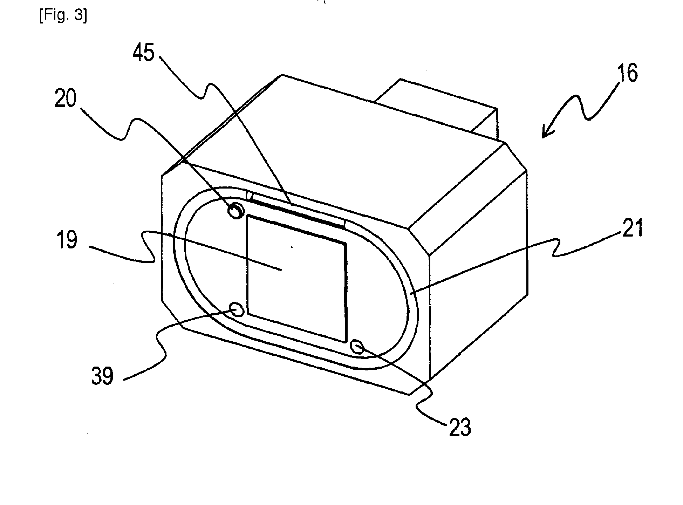

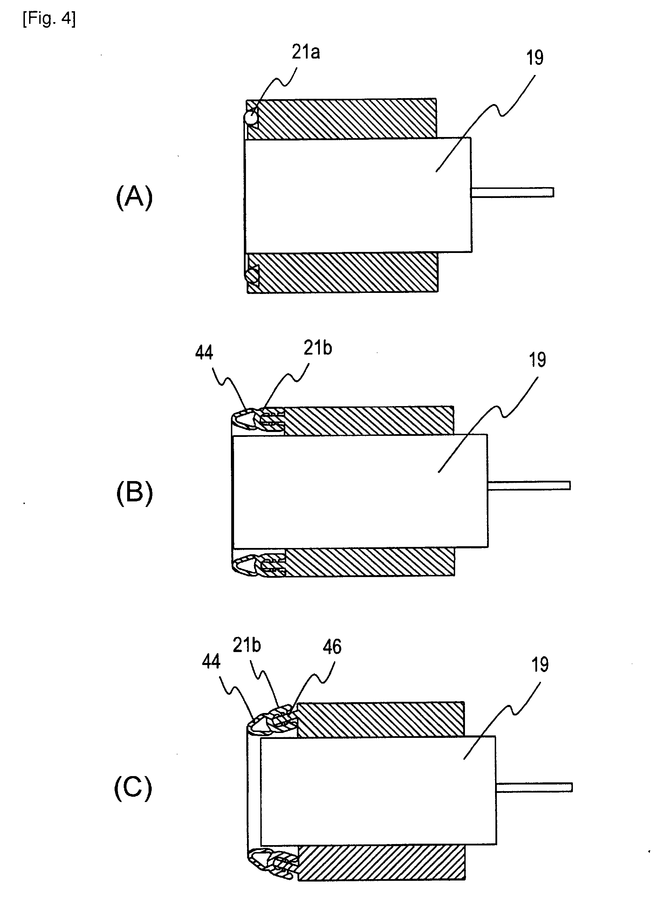

Next, a configuration of the acoustic wave probe is described in detail with reference to Figs. 3 and 4A to 4C. Fig. 3 is a perspective view of the acoustic wave probe. Figs. 4A to 4C are cross-sectional views in detail of the acoustic wave probe.

As illustrated in Fig. 3, the seal member 21 is provided on a surface on a measurement side of the acoustic wave probe 16 so as to enclose the periphery of the acoustic wave conversion unit (acoustic sensor) 19. The delivery port 23 for providing the lubricant and the retrieve port 39 for retrieving the lubricant are provided on a lower part of the space enclosed by the seal member 21 and the liquid level sensor 20 is provided on an upper part of the same. An opening 45 for letting air mixed in the lubricant out is formed on an upper part of the seal member 21. Packing made of a material such as rubber, a porous material (sponge), leather and resin may be used as the seal member 21.

Fig. 4A illustrates an example in which an O ring is used as a seal member 21a. A concave groove is formed around the acoustic wave conversion unit 19 and a circular O ring is provided in the concave groove. Lip packing, sponge packing and the like may also be used in place of the O ring. Fig. 4B illustrates an example in which a silicon trim (registered trademark) HR-06 from Hotty Polymer Inc. is used as a seal member 21b. A tip end 44 of the seal member 21b has a sharp angle and a hollow inside, so that the tip end 44 is crushed to exert excellent adhesiveness even when a pressure to the compression plate 17 is small. Also, the crush of the tip end 44 absorbs variation in distance between the compression plate 17 and the probe 16, so that the adhesiveness may be kept even when the compression plate 17 deforms by approximately 5 mm due to strain at the time of compression. For example, when parallelism between the compression plate 17 and the conversion unit 19 is 0.5 mm or less, the matching by the lubricant may be kept even when the conversion unit 19 is moved at 200 mm/sec with the crush of the tip end 44 of the seal member 21b by approximately 0.2 mm. Also, the bubble is not mixed from an interface between the seal member 21b and the compression plate 17. Fig. 4C illustrates a further preferable mode. In Fig. 4C, the tip end 44 of the seal member 21b is inclined by attaching the seal member 21b to an inclined portion 46 inclined toward the conversion unit 19. According to this configuration, it becomes easy to let the bubble out from between the conversion unit 19 and the compression plate 17 along the inclination when the bubble enters the lubricant.

### (Method for Measuring and Controlling)

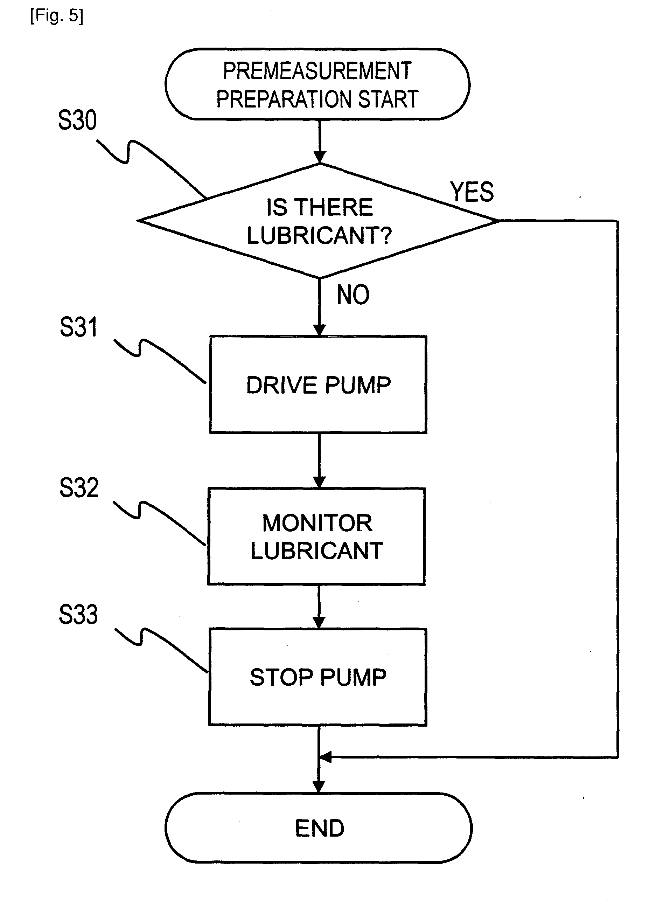

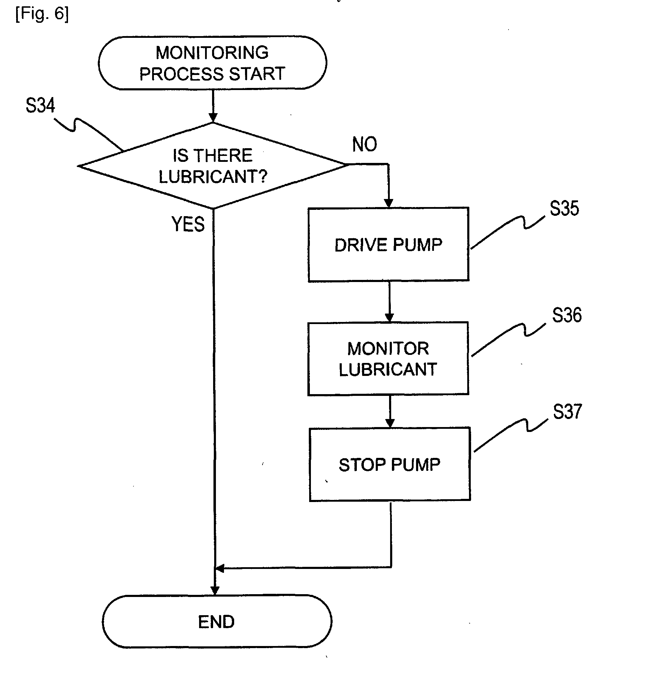

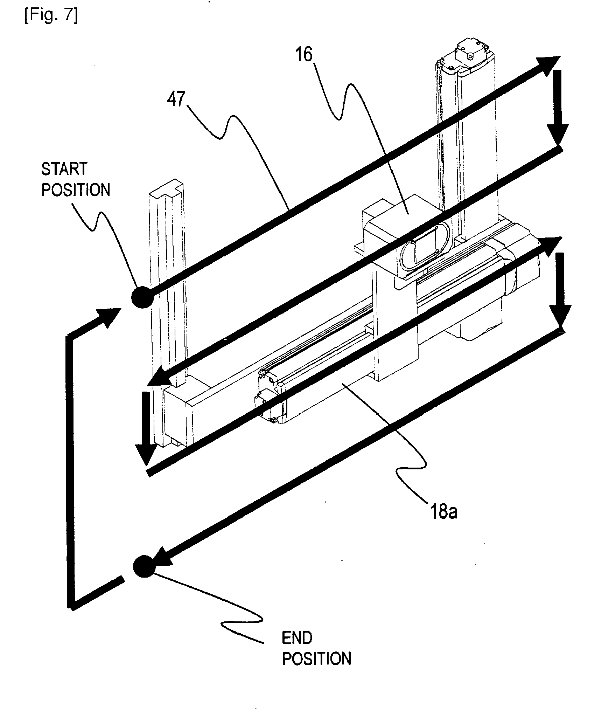

Next, a method for measuring and controlling by the apparatus of the present embodiment is described with reference to Figs. 5, 6 and 7. Fig. 5 is a flow chart illustrating a flow of a premeasurement preparation process. Fig. 6 is a flowchart illustrating a flow of a monitoring process of the lubricant during the measurement. Fig. 7 illustrates a trajectory diagram of the acoustic wave probe during the measurement.

A process to be described hereinafter is executed by a controlling unit 100 in Fig. 1. The controlling unit 100 is a controlling unitincluding a microcontroller or a computer for controlling the drive unit 18, the pump 25, the temperature control system 26, the valve 49 and the like. Information such as a result of detection by the lubricant monitoring unit and the temperature of the lubricant measured by the temperature control system 26 is input to the controlling unit 100 to be used to provide the lubricant and controlling the temperature.

As illustrated in Fig. 5, as the premeasurement preparation, the controlling unit 100 first confirms whether the space 22 is filled with the lubricant based on the result of detection by the lubricant monitoring unit (step S30). When it is confirmed that the space 22 is filled with the lubricant, the premeasurement preparation is completed. When it is confirmed that there is no lubricant in the space 22 in step S30, the controlling unit 100 drives the pump 25 to provide the lubricant into the space 22 (step S31). When the controlling unit 100 detects that the space 22 is filled with the lubricant from the result of detection by the lubricant monitoring unit (step S32), the controlling unit 100 stops the pump 25 (step S33). By the above-described premeasurement preparation process, the space 22 between the compression plate 17 and the acoustic wave conversion unit 19 may be filled with the appropriate amount of lubricant, and the preparation for performing excellent measurement is completed.

The controlling unit 100 always monitors whether the amount of the lubricant in the space 22 is appropriate also during the measurement. Specifically, the controlling unit 100 periodically repeatedly executes the process in Fig. 6. That is to say, when the controlling unit 100 imports the result of detection by the lubricant monitoring unit at a constant time interval anddetects that the space 22 is not filled with the lubricant (step S34), the controlling unit 100 drives the pump 25 to provide the lubricant (step S35). When the controlling unit 100 confirms that the space 22 is filled with the lubricant from the result of detection by the lubricant monitoring unit (step S36), the controlling unit 100 stops the pump 25 (step S37). By monitoring and controlling the amount of the lubricant in this manner, a state of the lubricant between the conversion unit 19 and the compression plate 17 may be kept appropriate and highly reliable measurement becomes possible. Also, since the pump 25 is not continuously driven, an effect of reducing electric noise and reducing consumed power may be obtained and noise of the pump, which disturbs patients, may be minimized.

An arrow 47 in Fig. 7 indicates a trajectory of the movement of the acoustic wave probe 16 at the time of the measurement. The probe 16 is located on an uppermost side end of a measurement range at an initial stage of the measurement. When the measurement is started, as indicated by the arrow 47, the probe 16 scans one line while moving in a horizontal direction, then moves downward by one line and scans a next line while moving in an opposite direction in the horizontal direction. By repeating this action, the lines are sequentially scanned one by one from top to bottom. The probe 16 returns to the start position when the probe 16 reaches the end position. When the bubble is mixed in the lubricant in the space 22, it is possible to let the bubble out from the opening 45 when the probe 16 moves from top to bottom.

The acoustic wave conversion unit 19 converts the received acoustic wave into an electric signal to output to an image generating unit 42. The image generating unit 42 generates an image representing information in the living body based on the signal. Meanwhile, since a conventionally known method may be used as a method for composing the information in the living body from the acoustic wave signal, a detailed description thereof will not be repeated. The image generated by the image generating unit 42 is output to a display system 43.

According to the configuration of the present embodiment described above, it is possible to prevent a trouble in providing the lubricant, appropriately keep the state (amount) of the lubricant between the acoustic wave conversion unit 19 and the compression plate 17 and quickly remove the bubble mixed in the lubricant. Therefore, the highly reliable acoustic wave measurement may be realized. Also, since the space 22 filled with the lubricant is formed by enclosing the periphery of the conversion unit 19 by the seal member 21, the adhesiveness between the conversion unit 19 and the compression plate 17 may be kept even when the probe 16 is rapidly moved and the stable measurement is possible. Also, since the lubricant is always present between the compression plate 17 and the conversion unit 19, occurrence of damage by friction may be prevented. Further, since a configuration to retrieve and circulate the lubricant leaking from the seal member 21 and the lubricant in the space 22 is provided, waste of the lubricant is eliminated, so that it is economical.

### <Second Embodiment>

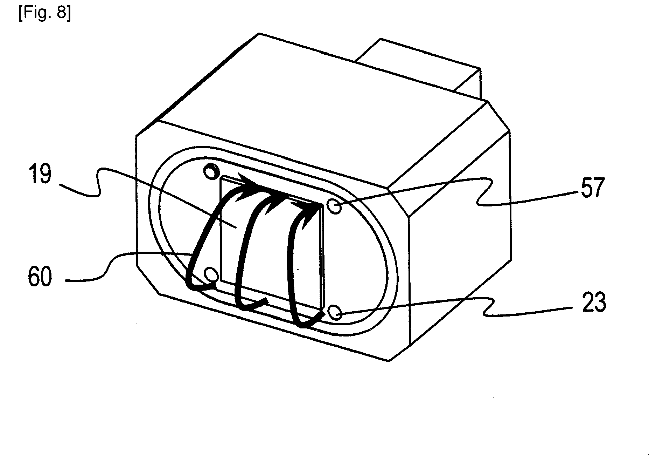

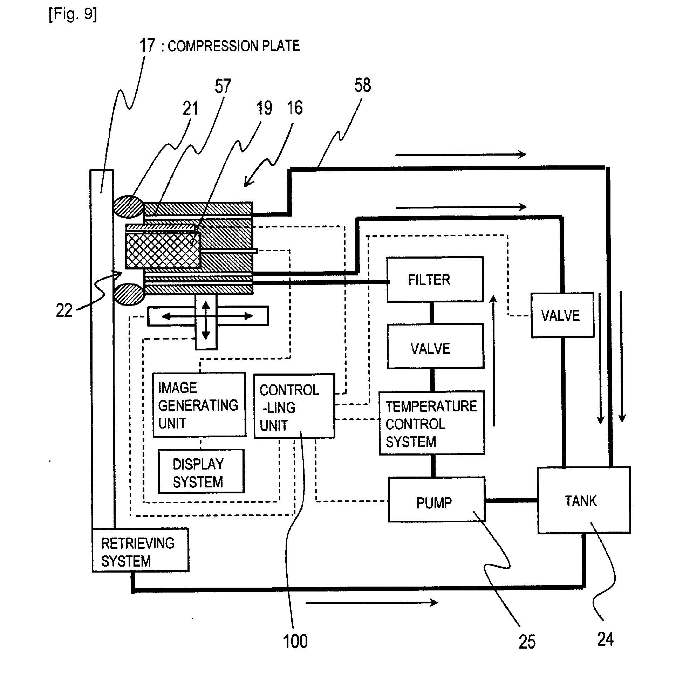

The acoustic wave measuring apparatus according a second embodiment is described with reference to Figs. 8 and 9. Fig. 8 is a perspective view of the acoustic wave probe of the present embodiment and Fig. 9 is a block diagram illustrating a schematic configuration of the acoustic wave measuring apparatus of the present embodiment. Although the opening to let the bubble out is provided on the upper part of the seal member in the first embodiment, the bubble is removed by a circulating unit for circulating the lubricant in the space formed by the seal member in the second embodiment.

The acoustic wave probe 16 in the present embodiment has a retrieve port 57 of the lubricant on an upper part of the space 22. The retrieve port 57 is connected to the tank 24 through a pipe 58. The configuration other than the above configuration is the same as that in Fig. 1 of the first embodiment.

As illustrated in Fig. 8, by retrieving the lubricant from the retrieve port 57, a flow of the lubricant from bottom to top as indicated by an arrow 60 is formed in the space 22. When the bubble is mixed in between the compression plate 17 and the conversion unit 19, the bubble may be removed by forming the flow 60 of the lubricant from bottom to top.

Although the method for measuring and controlling is substantially similar to that of the first embodiment, when it is required to intermittently remove the bubble mixed in the lubricant, for example, the pump 25 is continuously operated from the start of the measurement. When it is not required to continuously operate the pump 25, it is also possible to monitor the bubble by each transducer of the acoustic wave conversion unit 19 and drive the pump 25 when detecting the bubble to circulate the lubricant.

According to the configuration of the present embodiment as described above, the bubble mixed in the lubricant may be surely removed. Therefore, the reliability and stability of the measurement may be improved.

While the present invention has been described with reference to exemplary embodiments, it is to be understood that the invention is not limited to the disclosed exemplary embodiments.

This application claims the benefit of Japanese Patent Application No. 2009-258930, filed on November 12, 2009.

## Claims

1. An acoustic wave measuring apparatus, comprising:
a plate for pressing against a subject;
a probe that has an acoustic wave conversion unit for receiving an acoustic wave emitted from the subject through the plate to convert into an electric signal;
a seal member that is provided on the probe so as to enclose a periphery of the acoustic wave conversion unit, and that forms a space to hold lubricant between the plate and the acoustic wave conversion unit;
a providing unit that provides the lubricant into the space formed by the seal member; and **characterised by**
a lubricant monitoring unit that detects whether the space between the plate and the acoustic wave conversion unit is filled with the lubricant.

2. The acoustic wave measuring apparatus according to claim 1, wherein
when the lubricant monitoring unit detects that the space between the plate and the acoustic wave conversion unit is not filled with the lubricant, the providing unit provides the lubricant into the space.

3. The acoustic wave measuring apparatus according to claim 1, wherein
the acoustic wave conversion unit doubles as the lubricant monitoring unit.

4. The acoustic wave measuring apparatus according to claim 1, wherein
the lubricant monitoring unit is a liquid level sensor for detecting whether the space is filled with the lubricant to a position higher than the acoustic wave conversion unit.

5. The acoustic wave measuring apparatus according to claim 1, wherein
the seal member is packing made of rubber, a porous material, leather or resin.

6. The acoustic wave measuring apparatus according to claim 1, further comprising
a retrieving unit that retrieves the lubricant leaked out of the space from the seal member allowing the lubricant to circulate to the providing unit.

7. The acoustic wave measuring apparatus according to claim 1, wherein
the seal member includes an opening to let air mixed in the lubricant in the space out of the space.

8. The acoustic wave measuring apparatus according to claim 1, further comprising
a circulating unit that removes air mixed in the lubricant in the space by allowing the lubricant in the space to circulate.

9. The acoustic wave measuring apparatus according to claim 1, further comprising
a temperature controlling unit that controls a temperature of the lubricant provided into the space by the providing unit at a predetermined temperature.

10. The acoustic wave measuring apparatus according to any one of claims 1 to 9, wherein
a tip end of the seal member is inclined toward the acoustic wave conversion unit.

## Patentansprüche

1. Akustikwellenmessvorrichtung, mit:
einer Platte zum Drücken gegen ein Subjekt;
einer Sonde, die eine Akustikwellenumsetzeinheit zum Empfangen einer von dem Subjekt abgestrahlten Akustikwelle durch die Platte aufweist, zum Umsetzen in ein elektrisches Signal;
einem Dichtungselement, das an der Sonde bereitgestellt ist, um so einen Umfang der Akustikwellenumsetzeinheit zu umschließen, und das einen Raum bildet, um ein Schmiermittel zwischen der Platte und der Akustikwellenumsetzeinheit zu halten;
einer Bereitstelleinheit, die das Schmiermittel in den durch das Dichtungselement gebildeten Raum bereitstellt; und **gekennzeichnet durch**
eine Schmiermittelüberwachungseinheit, die erfasst, ob der Raum zwischen der Platte und der Akustikwellenumsetzeinheit mit dem Schmiermittel gefüllt ist.

2. Akustikwellenmessvorrichtung gemäß Anspruch 1, wobei
wenn die Schmiermittelüberwachungseinheit erfasst, dass der Raum zwischen der Platte und der Akustikwellenumsetzeinheit nicht mit dem Schmiermittel gefüllt ist, die Bereitstelleinheit das Schmiermittel in den Raum bereitstellt.

3. Akustikwellenmessvorrichtung gemäß Anspruch 1, wobei
die Akustikwellenumsetzeinheit sich als die Schmiermittelüberwachungseinheit doppelt.

4. Akustikwellenmessvorrichtung gemäß Anspruch 1, wobei
die Schmiermittelüberwachungseinheit ein Flüssigkeitspegelsensor ist, zum Erfassen, ob der Raum mit dem Schmiermittel zu einer Position höher als die Akustikwellenumsetzeinheit gefüllt ist.

5. Akustikwellenmessvorrichtung gemäß Anspruch 1, wobei
das Dichtungselement eine aus Gummi, einem porösen Material, Leder oder Harz hergestellte Dichtung ist.

6. Akustikwellenmessvorrichtung gemäß Anspruch 1, weiterhin mit
einer Zurückgewinnungseinheit, die das aus dem Raum geleckte Schmiermittel aus dem Dichtungselement zurückgewinnt, was dem Schmiermittel ermöglicht, zu der Bereitgestellungseinheit zu zirkulieren.

7. Akustikwellenmessvorrichtung gemäß Anspruch 1, wobei
das Dichtungselement eine Öffnung umfasst, um mit dem Schmiermittel vermischte Luft in den Raum außerhalb des Raums abzulassen.

8. Akustikwellenmessvorrichtung gemäß Anspruch 1, weiterhin mit
einer Zirkuliereinheit, die mit dem Schmiermittel in dem Raum vermischte Luft entfernt, durch Ermöglichen, dass das Schmiermittel in den Raum zirkuliert.

9. Akustikwellenmessvorrichtung gemäß Anspruch 1, weiterhin mit
einer Temperatursteuereinheit, die eine Temperatur des Schmiermittels, das durch die Bereitstelleinheit in den Raum bereitgestellt wird, auf eine vorbestimmte Temperatur steuert.

10. Akustikwellenmessvorrichtung gemäß einem der Ansprüche 1 bis 9, wobei
ein oberes Ende des Dichtungselements in Richtung der Akustikwellenumsetzeinheit geneigt ist.

## Revendications

1. Appareil de mesure d'onde acoustique, comprenant :
une plaque destinée à être pressée contre un sujet ;
une sonde qui a une unité de conversion d'onde acoustique destinée à recevoir une onde acoustique émise par le sujet à travers la plaque pour la convertir en un signal électrique ;
un élément d'étanchéité qui est prévu sur la sonde afin d'enfermer la périphérie de l'unité de conversion d'onde acoustique, et qui forme un espace pour contenir un lubrifiant entre la plaque et l'unité de conversion d'onde acoustique ;
une unité d'alimentation qui alimente le lubrifiant dans l'espace formé par l'élément d'étanchéité ; et **caractérisé par**
une unité de surveillance de lubrifiant qui détecte si l'espace entre la plaque et l'unité de conversion d'onde acoustique est rempli de lubrifiant.

2. Appareil de mesure d'onde acoustique selon la revendication 1, dans lequel
lorsque l'unité de surveillance de lubrifiant détecte que l'espace entre la plaque et l'unité de conversion d'onde acoustique n'est pas rempli de lubrifiant, l'unité d'alimentation alimente le lubrifiant dans l'espace.

3. Appareil de mesure d'onde acoustique selon la revendication 1, dans lequel
l'unité de conversion d'onde acoustique fait également office d'unité de surveillance de lubrifiant.

4. Appareil de mesure d'onde acoustique selon la revendication 1, dans lequel
l'unité de surveillance de lubrifiant est un capteur de niveau de liquide destiné à détecter si l'espace est rempli de lubrifiant à une position plus élevée que celle de l'unité de conversion d'onde acoustique.

5. Appareil de mesure d'onde acoustique selon la revendication 1, dans lequel
l'élément d'étanchéité est une garniture d'étanchéité réalisée en caoutchouc, en un matériau poreux, en cuir ou en résine.

6. Appareil de mesure d'onde acoustique selon la revendication 1, comprenant en outre
une unité de récupération qui récupère le lubrifiant qui s'échappe de l'espace à partir de l'élément d'étanchéité permettant au lubrifiant de circuler vers l'unité d'alimentation.

7. Appareil de mesure d'onde acoustique selon la revendication 1, dans lequel
l'élément d'étanchéité comporte une ouverture pour permettre à l'air mélangé dans le lubrifiant dans l'espace de s'échapper de l'espace.

8. Appareil de mesure d'onde acoustique selon la revendication 1, comprenant en outre
une unité de circulation qui élimine l'air mélangé dans le lubrifiant dans l'espace en permettant la circulation du lubrifiant dans l'espace.

9. Appareil de mesure d'onde acoustique selon la revendication 1, comprenant en outre
une unité de réglage de température qui règle une température du lubrifiant prévu dans l'espace par l'unité d'alimentation à une température prédéterminée.

10. Appareil de mesure d'onde acoustique selon l'une quelconque des revendications 1 à 9, dans lequel
une extrémité de pointe de l'élément d'étanchéité est inclinée vers l'unité de conversion d'onde acoustique.
